# EUROPEAN PATENT APPLICATION

(11) **EP 4 838 451 A1**
(43) Date of publication of application: **11.11.2026**
(21) Application number: 25191868.6
(22) Date of filing: 25.07.2025
(51) Int. Cl.: G06Q 30/0601, B01F 33/84, G06Q 50/04, G16C 20/70

(54) **A METHOD FOR REAL-TIME PRODUCTION OF PERSONALIZED SKIN CARE PRODUCTS**

(30) Priority: 08.05.2025 CN 202510584673
(71) Applicant: Bao, Jiayin, Toronto Ontario M2P1R5 (CA)
(72) Inventor: Bao, Jiayin, Toronto Ontario M2P1R5 (CA)
(74) Representative: Valet Patent Services Limited

(57) **Abstract**

Method for the real-time creation of customized skincare products, which includes the following steps: The user initiates the selected service via an interactive interface that collects spectral images and sensor data of their skin. This information is processed by AI to evaluate skin condition and develop a tailored skincare formula. Corresponding formula instructions are generated and sent to a robotic arm. Operating in a cleanroom, the robotic arm selects the necessary ingredients from a raw material storage unit, measures and blends them accurately to produce the final product, which is then filled into a container. Once payment is completed, the user can collect the product on-site. This method supports a real-time process integrating skin data collection, formula creation, product production, and sales, enabling on-the-spot pickup and meeting the need for rapid personalization.

## Description

### CROSS-REFERENCE TO RELATED APPLICATIONS

This application claims priority to Chinese Patent Application No. 202510584673.4, filed on May 08, 2025, which is hereby incorporated by reference in its entirety.

### TECHNICAL FIELD

The present invention pertains to the field of skincare product manufacturing technology, and more specifically, relates to a real-time production method for personalized skincare products.

### BACKGROUND

With the rapid development of the beauty and skincare industry, consumer demand for skincare products has become increasingly diversified and personalized.

Traditional formulation technologies for skincare products are no longer sufficient to meet market demands.

However, existing technologies still largely rely on experience-based or semi-automated research and development methods. Conventional formula design is often based on the empirical knowledge of R&D personnel and common ingredient combinations, lacking in-depth study and accurate prediction of the chemical interactions between skincare ingredients and skin components, as well as among the skincare ingredients themselves. Without the ability to anticipate potential chemical reactions, their extent, or the resulting products in advance, conflicts between ingredients may arise within a formula. This not only diminishes product efficacy but may also cause adverse effects on the skin, thereby undermining the scientific validity and effectiveness of the formulation.

Moreover, the current R&D processes for skincare formulations exhibit a low degree of automation and require significant manual intervention. For instance, during formulation trials, researchers must manually mix ingredients in various proportions and conduct numerous repetitive experiments to explore different formula combinations, while manually recording and analyzing the results. This approach consumes substantial time and human resources, leads to inefficiencies in data processing, and impedes timely responses to the diversified needs of the market. As consumers increasingly demand faster iteration and customization of skincare products, the long R&D cycles and high costs associated with existing technologies have become critical drawbacks, severely restricting industry development and innovation.

In addition, conventional skincare products currently rely on fixed formulations, which are unable to dynamically adjust ingredient proportions based on the user's real-time skin condition. Such static formulations fail to meet the evolving and dynamic demands of users. Furthermore, the lack of a retail model capable of generating and dispensing skincare products in real time based on individual skin conditions limits the feasibility of instant and adaptive production, rendering current systems inadequate for real-time customization and delivery.

To address these limitations, technical personnel in the field have proposed a real-time production method for personalized skincare products, aimed at enabling the efficient research and development of personalized skincare formulas through more scientific and comprehensive methodologies. This invention seeks to fundamentally improve the degree of personalization, scientific rigor, and market responsiveness of skincare products, thereby better satisfying modern consumer expectations.

### SUMMARY

To solve the above-mentioned technical problems, the present invention provides a real-time production method for personalized skincare products, addressing the deficiencies outlined in the background art.

The method comprises the following steps:
S1. The user selects the corresponding service via an interactive interface. A hyperspectral imaging device and sensor device are activated to acquire a spectral characteristic image of the skin and associated sensor data;
S2. The acquired spectral feature image and sensor data are subjected to AI analysis to determine the user's individual skin condition and generate a personalized skincare product formula;
S3. A formula instruction is generated based on the personalized skincare product formula, and the instruction is transmitted to a robotic arm;
S4. In a cleanroom environment, the robotic arm retrieves the corresponding ingredients from a raw material storage unit in accordance with the formula instruction. The ingredients are proportionally dispensed and mixed to produce the final product, which is then filled into a product container. Upon payment, the user may collect the product on-site.

Preferably, in Step S1, the interactive interface is an intelligent mirror terminal integrated with a camera and touchscreen;The user selects the type of service through the interface and activates the hyperspectral imaging device and sensor device to acquire the spectral characteristic image and sensor data of the skin;The spectral characteristic image includes information on wrinkles, capillary redness, and hydration status; the sensor data includes pH value and sebum level measurements.

Preferably, in Step S2, AI analysis is performed on the spectral characteristic image and sensor data to generate a skin ID profile with quantifiable indicators.

A chemical interaction prediction model is used to cross-reference the skin ID profile with a bioactive compound library, thereby generating chemical interaction prediction results.

Based on the prediction results, a stability optimization algorithm is applied to adjust the skincare formula and obtain an optimized formulation.

A multi-factor stability evaluation model is then used to comprehensively assess the optimized formulation and generate the final skincare product formula.

Preferably, the step of cross-referencing the skin ID profile with the bioactive compound library through the chemical interaction prediction model to generate chemical interaction prediction results comprises:
Using molecular structures present in the skin ID profile, Morgan fingerprints are applied to iteratively expand the molecular structures and generate fixed-length *Nᵤ* binary molecular feature vectors *Fᵤ*;
The molecules of skincare ingredients in the bioactive compound library are also encoded. For any molecule *M_{bio}* in the library, a binary feature vector *F_{bio}* is obtained using Morgan fingerprint encoding, and *F_{bio}* ∈ *R^{Nᵤ}*;
The molecular feature vector *Fᵤ* encoded in the skin ID profile is compared with each molecular feature vector *F_{bio}* in the bioactivity library. The characteristic vectors of the molecules are combined to form input pairs (*Fᵤ*,*F_{bio}*); for n molecules in the bioactive library, several n input pair sets *Fᵢₙₚᵤₜ* = [*Fᵤ,F_{bio}*] ∈ *R*^{2*N*ᵤ} are obtained;
Using a deep learning-based chemical interaction prediction model, the input pair (*Fᵤ,F_{bio}*) is spliced into *Fᵢₙₚᵤₜ* = [*Fᵤ,F_{bio}*] ∈ R²_{Nᵤ}, after calculation by the chemical interaction prediction model, an output vector Y is obtained, and input *Y* E *R^{Dₒᵤₜₚᵤₜ}*, where *Dₒᵤₜₚᵤₜ* is the output dimension, which is set according to the preset prediction task; the prediction task includes reaction possibility prediction and reaction product prediction;
Based on the output vector Y, the prediction task is analyzed to generate a chemical interaction prediction.

Preferably, the skin care formula is adjusted according to the chemical interaction prediction result by a stability optimization algorithm to obtain an optimized formula, comprising:
Based on the chemical interaction prediction results, a molecular dynamics simulation and experimental data were combined to construct
The stability evaluation model is used to obtain the formula stability: $S \left(X\right) = {\sum }_{i = 1}^{n - 1} {\sum }_{j = i + 1}^{n} \left(1-{P}_{ij}\right) {K}_{ij} {x}_{i} {x}_{j}$, and *X* = [*x*₁,*x*₂,...,*xₙ*] represents the ratio vector of n ingredients in the skin care formula, *Pᵢⱼ* is the probability of the reaction between ingredient*ⁱ* and ingredient *j*, and *Kᵢⱼ* is the stability coefficient of the reaction product;
Based on the efficacy data of the ingredients in the bioactive library, the efficacy prediction model is used to predict the efficacy of the ingredients according to the formulation requirements.

Calculate the weight of each effect, and sum them up to get the total skin care effect: $E \left(X\right) = {\sum }_{k = 1}^{m} {a}_{k} {E}_{k} \left(X\right)$, where m is the number of effect types, *aₖ* is the weight of the *k* effect, and *Eₖ*(*X*) is the calculation function of the *k* effect;
According to the market price of ingredients, calculate the formula cost: $C \left(X\right) = {\sum }_{i = 1}^{n} {p}_{i} {x}_{i}$, where *pᵢ* is the unit of ingredient *i* price;
According to the formula stability *S*(*X*), total skin care efficacy *E*(*X*) and formula cost *C*(*X*), the multi-objective $F \left(X\right) = {ω}_{1} S \left(X\right) + {ω}_{2} E \left(X\right) + {ω}_{3} C \left(X\right)$
Among them, *ω*₁, *ω*₂, *ω*₃, which are used to corresponding weight coefficients, which are used to balance the importance of different objectives; component ratio 0 ≤ x*ᵢ* ≤ 1, ${\sum }_{i = 1}^{n} {x}_{i} = 1$; total skin care efficacy is *E*(*X*) ≥ *θ*; *θ* is the preset minimum skin care efficacy threshold;
Through the particle swarm optimization algorithm, iterative optimization is performed until the stopping condition is met, and the global optimal position is output as the optimized formula.

Preferably, in step S3, a formula instruction is generated according to the personalized skin care product formula, and the formula instruction is sent to a robotic arm, the robotic arm adopts a 6-axis robotic arm, uses a peristaltic pump as a power source for driving, and controls the robotic arm in combination with a program set by the formula instruction;
The mechanical arm is provided with force feedback to prevent droplets from splashing;
The robot arm is also equipped with a sterile micro-nozzle for taking the raw material ingredients and sterilizing them after use.

Batch replacement is performed to prevent cross contamination.

Preferably, in step S4, in a clean room environment, the robot arm performs the following steps according to the recipe instruction:
Take the corresponding formula ingredients from the raw material warehouse, mix them according to the proportion to make the finished product, and fill the finished product into the product container;
The clean room environment is ISO5-7 clean room standard, with dynamic particle count ≤3,52000/m²;
The raw material warehouse adopts a temperature-controlled raw material warehouse, and the temperature control range is -20°C to 40°C;
The raw material warehouse is also equipped with an RFID tag cartridge for tracking the validity period of the raw materials.

When it is close to expiration, an automatic alarm will be issued.

Preferably, in step S4, the robot arm performs the following operations according to the formula instruction obtained in step S3:
Set up the program to complete the operations of taking raw materials from the raw material warehouse, mixing and filling according to proportion;

The motion path of the robot arm is represented by three-dimensional coordinates (*x*,*y*,*z*).

There are n kinds of raw material components, among which the i raw material component moves from the raw material bin position (*x*_{*i*1},*y*_{*i*1},*z*_{*i*1}) to the packing position (*x*_{*i*2},*y*_{*i*2},*z*_{*i*2}), then to the mixing position (*x*_{*i*3},*y*_{*i*3},*z*_{*i*3}), and finally to the filling position (*x*_{*i*4},*y*_{*i*4},*z*_{*i*4});
Use the following formula to calculate the gripping force of the robot arm *Fᵢ*: ${F}_{i} = {σ}_{1} {Q}_{i} + {σ}_{2} {ρ}_{i}$
Among them, *σ*₁, *σ*₂ are all constants, *Qᵢ* is the content of the i raw material, *ρᵢ* is the density of the i raw material;
Calculate the mixing time T using the following formula: $T = {σ}_{3} {Q}_{θ} + {σ}_{4}$
Among them, *σ*₃, *σ*₄ are constants, and 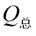 is the total content of all raw materials;
The motion path, grasping force and mixing time are integrated into execution instructions, and the formula instructions are recognized by the robotic arm and operations are performed according to the corresponding execution instructions.

Compared with the prior art, the present invention has the following beneficial effects:
1.The present invention provides an end-to-end solution-from the acquisition of user skin data, to the generation of personalized formulations, and finally to real-time production-all centered around individual differences. It meets consumers' personalized skincare needs by employing AI-based analysis, a variety of scientific models, and algorithmic optimization to formulate products. These processes fully account for chemical interactions among ingredients, thereby enhancing product efficacy, avoiding component conflicts, and ensuring quality and safety. Robotic arm automation, combined with a cleanroom environment, enables high production efficiency and rapid response to market demand.
2.The invention realizes a real-time workflow encompassing skin data acquisition, formulation generation, production, and sales, allowing for on-site product collection. It meets the demand for instant customization, setting it apart from conventional skincare products that rely on fixed formulas and are unable to respond quickly to market changes.

### BRIEF DESCRIPTION OF DRAWINGS

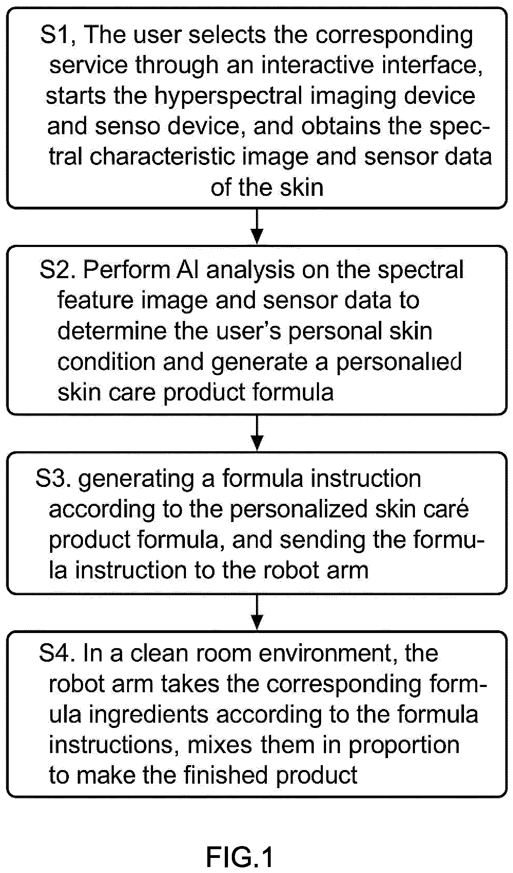

FIG. 1 is a flowchart illustrating the real-time production method for personalized skincare products according to the present invention.

### DESCRIPTION OF EMBODIMENTS

The following provides a more detailed description of the embodiments of the present invention with reference to the accompanying drawings and examples. These embodiments are intended to illustrate the invention but not to limit its scope.

As shown in FIG. 1,

Embodiment: The invention provides a real-time production method for personalized skincare products, comprising the following steps:
S1. The user selects the corresponding service via an interactive interface, activates a hyperspectral imaging device and a sensor device to acquire a spectral characteristic image of the skin and related sensor data;

Specifically, the interactive interface is an intelligent mirror terminal integrated with a camera and a touchscreen. Through the interactive interface, the user selects the type of service and initiates the hyperspectral imaging and sensor devices, which capture the spectral characteristic image and sensor data of the user's skin.

The spectral characteristic image includes information on wrinkles, capillary redness, and moisture levels; the sensor data includes pH values and sebum levels.

The user selects a service via an intelligent mirror terminal integrated with a camera and touchscreen, and activates the hyperspectral imaging device and sensor device to acquire spectral characteristic images-including data on wrinkles, capillary redness, and moisture condition-as well as sensor data such as pH value and sebum levels. This enables the resulting skincare product formulation to be highly tailored to the user's actual skin condition.

S2. The spectral characteristic image and sensor data are subjected to AI analysis to determine the user's individual skin condition and generate a personalized skincare formulation.

Through AI analysis, a skin ID profile with quantifiable indicators is generated based on the spectral characteristic image and sensor data.

A chemical interaction prediction model is then used to cross-reference the skin ID profile with a bioactive compound library, generating a chemical interaction prediction result.

Based on the molecular structures in the skin ID profile, Morgan fingerprints are applied to iteratively expand each molecular structure, thereby generating a fixed-length *Nᵤ* binary molecular feature vector *Fᵤ.*

The molecules of skincare ingredients *M_{bio}* in the bioactive compound library are encoded as well. For any given molecule in the library, the Morgan fingerprint encoding is applied to generate a corresponding binary molecular feature vector *F_{bio}* and *F_{bio}* ∈ *R^{Nᵤ}.*

The encoded molecular feature vectors *Fᵤ* from the skin ID profile are then paired with the feature vectors *F_{bio}* of each molecule in the bioactive compound library to form input pairs (*Fᵤ,F_{bio}*)*.* For n molecules in the bioactive compound library, a set of n input pairs $\left\{\left({F}_{u}, {F}_{bio}^{1}\right),\left({F}_{u}, {F}_{bio}^{2}\right),…,\left({F}_{u}, {F}_{bio}^{n}\right),\right\}$ is obtained.

A deep learning-based chemical interaction prediction model is then used to concatenate each input pair (*Fᵤ,F_{bio}*) into a single vector *Fᵢₙₚᵤₜ* = [*Fᵤ,F_{bio}*] ∈ R²_{Nᵤ}, which is passed through the model to compute an output vector Y, and *Y* ∈ *R^{Dₒᵤₜₚᵤₜ}*; where *Dₒᵤₜₚᵤₜ* is the output dimension, which is set according to the preset prediction task; the prediction task includes reaction possibility prediction and reaction product prediction; Based on the output vector Y, the prediction task is analyzed to generate the chemical interaction prediction results.

According to the chemical interaction prediction results, the skin care formula is adjusted through the stability optimization algorithm to obtain the optimized formula; according to the chemical interaction prediction results, the stability evaluation model constructed by combining molecular dynamics simulation and experimental data is obtained to obtain the formula stability: $S \left(X\right) = {\sum }_{i = 1}^{n - 1} {\sum }_{j = i + 1}^{n} \left(1-{P}_{ij}\right) {K}_{ij} {x}_{i} {x}_{j}$, where *X* = [*x*₁,*x*₂,...,*xₙ*] represents the ratio vector of n ingredients in the skin care formula, *Pᵢⱼ* is the probability of reaction between ingredients *i* and ingredients *j*, and *Kᵢⱼ* is the stability coefficient of the reaction product;

Based on the efficacy data of the ingredients in the bioactive library, the total skin care efficacy is obtained by weighted summation based on the efficacy prediction model and the weights of each efficacy set according to the formula requirements: $E \left(X\right) = {\sum }_{k = 1}^{m} {a}_{k} {E}_{k} \left(X\right)$, where m is the number of efficacy types, *aₖ* is the weight of the *k* efficacy, and *Eₖ*(*X*) is the calculation function of the *k* efficacy;

According to the market price of ingredients, calculate the formula cost: $C \left(X\right) = {\sum }_{i = 1}^{n} {p}_{i} {x}_{i}$, where *Pᵢ* is the unit price of the ingredient *i*;

According to the formula stability *S*(*X*), total skin care efficacy *E*(*X*) and formula cost *C*(*X*), the multi-objective function is obtained:
$F \left(X\right) = {ω}_{1} S \left(X\right) + {ω}_{2} E \left(X\right) + {ω}_{3} C \left(X\right)$

Among them, *ω*₁, *ω*₂, *ω*₃ are corresponding weight coefficients used to balance the importance of different goals; in the ingredient ratio, 0 ≤ *xᵢ* ≤ 1, ${\sum }_{i = 1}^{n} {x}_{i} = 1$; the total skin care effect *E*(*X*) ≥ *θ*, and *θ* is the preset minimum skin care effect threshold;
A particle swarm optimization algorithm is employed to iteratively optimize the formulation until a termination condition is met. The globally optimal position is output as the optimized formulation.

A multi-factor stability evaluation model is used to comprehensively assess the optimized formulation and to generate the final skincare formula.

By performing AI analysis on the acquired data to generate a skin ID profile, and subsequently applying a series of procedures-including chemical interaction prediction models and stability optimization algorithms-the final skincare formula is obtained. Through the use of scientific models and algorithms, the value of the data is deeply mined. The formulation process fully considers the chemical interactions between skincare ingredients and skin components, as well as among the skincare ingredients themselves, thereby optimizing the stability, efficacy, and cost of the formulation, and ensuring its scientific validity, effectiveness, and personalization.

S3. A formulation instruction is generated based on the personalized skincare formula and transmitted to a robotic arm.

The robotic arm is a six-axis robotic system driven by a peristaltic pump as the power source. The robotic movement is controlled in accordance with the formulation instruction through programmed execution.

The robotic arm is equipped with force feedback functionality to prevent liquid droplet splashing.

It is further equipped with sterile micro-nozzles, which are used to dispense raw material ingredients. These micro-nozzles are replaced in batches after use to prevent cross-contamination.

By converting the personalized skincare formulation into command instructions and sending them to a six-axis robotic arm-driven by a peristaltic pump and equipped with force feedback and sterile micro-nozzles-the robotic arm is able to execute formulation instructions with high precision. The force feedback mechanism prevents splashing of liquid droplets, while the sterile micro-nozzles enable hygienic ingredient dispensing and batch replacement, thereby ensuring product quality and safety.

S4. In a cleanroom environment, the robotic arm retrieves the corresponding formulation ingredients from the raw material warehouse based on the formulation instruction. The ingredients are proportionally dispensed and mixed to produce the final product, which is then filled into a product container. Upon payment, the user collects the product on-site.

Specifically, in a cleanroom environment, the robotic arm executes the formulation instruction by retrieving the required ingredients from the raw material warehouse, proportionally dispensing and mixing them to produce the final product, which is then filled into a designated product container.

In-bottle mixing is carried out using microfluidic vortex technology, achieving uniform blending at a vortex speed of 2000 rpm, with a mixing uniformity exceeding 99%.

The cleanroom environment complies with ISO Class 5 to Class 7 standards, with dynamic particle counts not exceeding 3,520,000 particles/m³, meeting GMP (Good Manufacturing Practice) requirements.

The raw material warehouse utilizes a temperature-controlled system, with a temperature regulation range from -20°C to 40°C.

In addition, the raw material warehouse is equipped with RFID-labeled cartridges to track the shelf life of raw materials. When a batch of raw materials is nearing expiration, the system triggers an automatic alert.

The robotic arm, based on the formulation instructions obtained in Step S3, follows a pre-set program to complete ingredient retrieval from the warehouse, proportional dispensing, mixing, and final filling operations.

The motion path of the robot arm is represented by three-dimensional coordinates (*x*,*y*,*z*). When the personalized skin care formula contains several raw material ingredients, for the first raw material ingredient, the robot arm moves from the raw material bin position (*x*_{*i*1},*y*_{*i*1},*z*_{*i*1}) to the filling position (*x*_{*i*2},*y*_{*i*2},*z*_{*i*2}), then to the mixing position (*x*_{*i*3},*y*_{*i*3},*z*_{*i*3}), and finally to the filling position (*x*_{*i*4},*y*_{*i*4},*z*_{*i*4});
Use the following formula to calculate the gripping force F of the robot arm:
   ${F}_{i} = {σ}_{1} {Q}_{i} + {σ}_{2} {ρ}_{i}$
Among them, *σ*₁, *σ*₂ are all constants, *Qᵢ* is the content of the *i* raw material, and *ρᵢ* is the density of the *i* raw material;
Calculate the mixing time T using the following formula: 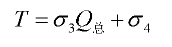
Among them, *σ*₃, *σ*₄ are constants, and 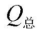 is the total content of all raw materials;
The motion path, gripping force and mixing time are integrated into execution instructions, and the robot arm recognizes the formula instructions and operates according to the corresponding execution instructions.

In an ISO Class 5 to 7 cleanroom environment, the robotic arm retrieves raw materials from the temperature-controlled raw material warehouse according to the formulation instructions. It performs proportional dispensing, mixing, and filling operations. The robotic system calculates gripping force and mixing duration based on specific formulas, and utilizes microfluidic vortex technology to achieve homogeneous mixing.

Operating under stringent environmental standards and with precise control parameters, the system ensures that the final product maintains stable and uniform quality. The entire process is efficient and capable of meeting user demands in real time.

As illustrated above, this production method breaks away from traditional experience-based research and development, leveraging advanced detection technologies and AI analysis to deeply mine skin data and generate precise, personalized formulations-rather than relying on fixed formulations or basic empirical blending.

The use of robotic automation minimizes manual intervention, reduces error rates and contamination risks, and significantly enhances both production efficiency and formulation consistency. This stands in stark contrast to traditional customized services that rely on manual weighing and mixing.

The real-time production and sales model implemented herein enables a fully integrated workflow-from skin data acquisition and formulation generation to product manufacturing and sales-with on-site pickup, thereby meeting the demand for instant customization. This approach distinguishes itself from conventional skincare product models, which are based on fixed formulas and lack the ability to respond rapidly to dynamic market needs.

### Practical Case Study:

A certain brand's anti-acne product caused barrier damage in sensitive-skin users due to excessive dryness.

Clinical data indicated that 23% of users reported adverse effects.

Manual Operation Introduces Error and Contamination Risks:
Existing customized services (e.g., in-pharmacy compounding) require manual weighing and mixing, which introduces a risk of cross-contamination (e.g., microbial overgrowth caused by non-sterilized instruments).

Experimental data showed that manual compounding had an error margin of ±8%, whereas the robotic system maintained a precision of ±0.5%.

### Market Limitation:

Commercially available skincare products lack the ability to dynamically optimize formulations based on post-use skin condition changes (e.g., seasonal dryness or acne flare-ups due to hormonal fluctuations).

### User Survey:

78% of consumers expressed a desire for skincare products that could "adaptively
improve skin problems.
Experimental Scenario: Retail Deployment
Hardware Configuration:
Cleanroom dimensions: 2m × 1.5m × 2m (L × W × H);
Ingredient library capacity: 50 active ingredients (e.g., retinol, vitamin C, peptides);
Operational Workflow:
The user scans their skin;
The AI system generates a diagnostic report (including metrics such as TEWL and erythema index);
The system automatically matches a personalized formula(e.g., 2% hyaluronic acid + 1% ceramide + 0.5% retinol);
The robotic arm dispenses and mixes the formulation, producing the final product within 5 minutes.

### Experimental Data:

Compared to traditional products, user satisfaction increased by 42% (N = 500, p < 0.01).

### Production Efficiency:

Each production cycle ≤ 5 minutes, with a daily output capacity of up to 200 units,
representing a 10-fold increase in efficiency compared to manual compounding.

The system achieved a 100% pass rate in microbiological testing, in accordance with the Technical Specifications for the Safety of Cosmetics.

Additionally, a long-term tracking and optimization plan may be configured. A mobile application is pushed to the user via the interactive interface. The user uploads weekly skin scan data through the mobile application. The intelligent product terminal compares the new data with historical records and automatically adjusts the formulation accordingly.

For example: if persistent dryness is detected, the hyaluronic acid concentration may be increased from 2% to 3.5%;

If TEWL (Transepidermal Water Loss) is measured at a low value such as 8g/m²/h, the ceramide ratio may be decreased while antioxidant components (e.g., 0.2% resveratrol) are increased.

The system also displays a "Skin Health Index" and a predicted improvement curve on the user's mobile device, such as "15% reduction in pore size over 8 weeks", thus enabling ongoing formula optimization based on updated skin data.

Through the continuous collection of skin data via the mobile application and intelligent adjustment of formulations, dynamic product optimization is realized. This stands in stark contrast to traditional commercial skincare products, which lack the capability to adapt formulations based on post-use skin condition changes, marking a significant advancement.

Importantly, it should be noted that the structures and arrangements described in the various exemplary embodiments of the present application are illustrative in nature only. Although several embodiments have been described in detail herein, those skilled in the art will readily recognize that various modifications and substitutions may be made without departing from the novel teachings and advantages disclosed herein.

Accordingly, changes may be made in the design, operational state, and configuration of the exemplary embodiments without departing from the scope of the present invention. The present invention should therefore not be construed as being limited to any specific embodiment, but rather as encompassing all modifications that fall within the scope of the appended claims.

Furthermore, in order to provide a concise description of the exemplary embodiments, not all features of actual implementations are described in detail-specifically, those features that are not directly relevant to the best mode currently contemplated for carrying out the invention, or that are not essential to the realization of the invention.

It should be understood that, in the development of any actual embodiment, numerous implementation-specific decisions must be made to achieve the developers' specific goals. Such development efforts may be complex and time-consuming; however, for those skilled in the art, the process will be a matter of routine engineering and design, without requiring undue experimentation, especially when guided by the teachings of this disclosure.

It should be noted that the foregoing embodiments are merely illustrative of the technical solutions of the present invention and are not intended to be limiting. Although the present invention has been described in detail with reference to preferred embodiments, those of ordinary skill in the art will understand that various modifications or equivalent substitutions may be made to the technical solutions disclosed herein without departing from the spirit and scope of the invention. All such modifications and equivalents shall fall within the scope of the appended claims.

## Claims

1. A method for real-time production of personalized skin care products, **characterized in that** it comprises the following steps:
S1. The user selects the corresponding service through the interactive interface and starts the hyperspectral imaging device and sensor device.
Acquire spectral characteristic images and sensor data of skin;
S2, performing AI analysis on the spectral characteristic image and sensor data to determine the user's personal skin condition,
Generate personalized skin care product formulations;
S3, generating a formula instruction according to the personalized skin care product formula, and sending the formula instruction to the machine
in the arm;
S4. In a clean room environment, the robot arm takes the corresponding formula ingredients from the raw material warehouse according to the formula instructions, mixes them in proportion to obtain the finished product, and fills the finished product into the product container. The user picks up the product on site after completing the payment.

2. A method for real-time production of personalized skin care products as claimed in claim 1, **characterized in that**:
In step S1, the interactive interface is an intelligent mirror terminal integrated with a camera and a touch screen. The user selects a service type through the interactive interface and starts a hyperspectral imaging device and a sensor device to obtain a spectral characteristic image and sensor data of the user's skin; the spectral characteristic image includes wrinkle information, red blood streak information and moisture status information; the sensor data includes pH value data and sebum data.

3. A method for real-time production of personalized skin care products as claimed in claim 1, **characterized in that**:
In step S2, AI analysis is performed on the spectral feature image and sensor data to generate a skin ID profile with quantifiable indicators;
Cross-referencing the skin ID profile with the bioactivity library through a chemical interaction prediction model to generate a chemical interaction prediction result;
According to the chemical interaction prediction results, the skin care formula is adjusted through the stability optimization algorithm.
After adjustment, the optimized formula is obtained;
The optimized formula is comprehensively evaluated through a multi-factor stability evaluation model to generate a final skin care formula.

4. A method for real-time production of personalized skin care products as claimed in claim 3, **characterized in that**:
The skin ID profiles were cross-referenced with bioactive libraries through chemical interaction prediction models.
Generate chemical interaction predictions, including:
Based on the molecules in the skin ID profile, the Morgan fingerprint is used to iteratively expand the molecular structure.
Expand and generate a fixed-length *Nᵤ* binary molecular feature vector *Fᵤ*;
Encode the skin care ingredient molecules in the bioactive library, and for any molecule *M_{bio}* in the library, the characteristic vector *F_{bio}* of each molecule is obtained by Morgan fingerprint encoding, and *F_{bio}* ∈ *R^{Nᵤ};*
The molecular feature vector *Fᵤ* is combined with the feature vector *F_{bio}* of each molecule in the bioactive library to form an input pair (*Fᵤ,F_{bio}*); for *n* molecules in the bioactive library, *n* input pair sets $\left\{\left({F}_{u}, {F}_{bio}^{1}\right),\left({F}_{u}, {F}_{bio}^{2}\right),…,\left({F}_{u}, {F}_{bio}^{n}\right),\right\}$ are obtained;
The chemical interaction prediction model based on deep learning is used to splice the input pair (*Fᵤ*,*F_{bio}*) into *Fᵢₙₚᵤₜ* = [*Fᵤ*,*F_{bio}*] ∈ R²_{Nᵤ}. After calculation by the chemical interaction prediction model, the output vector *Y* is obtained, and *Y* ∈ *R^{Dₒᵤₜₚᵤₜ}*, where *Dₒᵤₜₚᵤₜ* is the output dimension, which is set according to the preset prediction task; The prediction tasks include reaction possibility prediction and reaction product prediction;Based on the output vector Y, the prediction task is analyzed to generate a chemical interaction prediction result.

5. A method for real-time production of personalized skin care products according to claim 3, **characterized in that**: adjusting the skin care formula by a stability optimization algorithm according to the chemical interaction prediction result to obtain an optimized formula comprises:
According to the chemical interaction prediction results, the stability evaluation model constructed by combining molecular dynamics simulation and experimental data is obtained to obtain the formula stability: $S \left(X\right) = {\sum }_{i = 1}^{n - 1} {\sum }_{j = i + 1}^{n} \left(1-{P}_{ij}\right) {K}_{ij} {x}_{i} {x}_{j}$, where *X* = [*x*₁,*x*₂,...,*xₙ*] represents the ratio vector of n ingredients in the skin care formula, *Pᵢⱼ* is the probability of reaction between ingredients *i* and *j,* and *Kᵢⱼ* is the stability coefficient of the reaction product;
Based on the efficacy data of the ingredients in the bioactive library, the total skin care efficacy is obtained by weighted summation according to the efficacy weights set according to the formula requirements through the efficacy prediction model: $E \left(X\right) = {\sum }_{k = 1}^{m} {a}_{k} {E}_{k} \left(X\right)$, where *m* is the number of efficacy types, *aₖ* is the weight of the *k* efficacy, and *Eₖ*(*X*) is the calculation function of the *k* efficacy;
According to the market price of ingredients, calculate the formula cost: $C \left(X\right) = {\sum }_{i = 1}^{n} {p}_{i} {x}_{i}$, where *pᵢ* is the unit price of the ingredient *i*:
According to the formula stability *S(X),* total skin care efficacy *E(X)* and formula cost *C(X),* the multi-objective: $F \left(X\right) = {ω}_{1} S \left(X\right) + {ω}_{2} E \left(X\right) + {ω}_{3} C \left(X\right)$
Among them, *ω*₁, *ω*₂, *ω*₃ is the corresponding weight coefficient, which is used to balance the importance of different goals; component ratio 0 ≤ *xᵢ* ≤ 1, ${\sum }_{i = 1}^{n} {x}_{i} = 1$; the total skin care effect *E*(*X*) ≥ *θ* is 2 days, where *θ* is the preset minimum skin care effect threshold;
Through the particle swarm optimization algorithm, iterative optimization is performed until the stopping condition is met, and the global optimal position is output as the optimized formula.

6. A method for real-time production of personalized skin care products according to claim 1, **characterized in that**: in the step S3, a formula instruction is generated according to the formula of the personalized skin care products, and the formula instruction is sent to a robotic arm, the robotic arm is a 6-axis robotic arm, driven by a peristaltic pump as a power source, and the robotic arm is controlled by a program set in combination with the formula instruction;
The mechanical arm is provided with force feedback to prevent droplets from splashing;
The robotic arm is also equipped with a sterile micro-nozzle for taking raw material ingredients and performing batch replacement after use to prevent cross contamination.

7. A method for real-time production of personalized skin care products as claimed in claim 6, **characterized in that**: in the step S4, in a clean room environment, the robot arm takes the corresponding formula ingredients from the raw material warehouse according to the formula instruction, mixes them in proportion to obtain the finished product, and fills the finished product into the product container;
The clean room environment is ISO 5-7 clean room standard, with a dynamic particle count of ≤3,52000/m³;
The raw material warehouse adopts a temperature-controlled raw material warehouse, and the temperature control range is -20°C to 40°C;
The raw material warehouse is also equipped with an RFID tag cartridge for tracking the validity period of the raw materials.
When it is close to expiration, an automatic alarm will be issued.

8. A method for real-time production of personalized skin care products as claimed in claim 7, **characterized in that**: in the step S4, the robot arm completes the operations of taking raw materials from the raw material warehouse, mixing and filling according to the proportion according to the formula instruction obtained in step S3 and in accordance with the set program;
The motion path of the robot arm is represented by three-dimensional coordinates (*x*,*y*,*z*). When the personalized skin care formula contains several raw material ingredients, for the first raw material ingredient, the robot arm moves from the raw material bin position (*x*_{*i*1},*y*_{*i*1},*z*_{*i*1}) to the filling position (*x*_{*i*2},*y*_{*i*2},*z*_{*i*2}), then to the mixing position (*x*_{*i*3}*,y*_{*i*3},*z*_{*i*3}), and finally to the filling position (*x*_{*i*4},*y*_{*i*4},*z*_{*i*4});
Use the following formula to calculate the gripping force F of the robot arm: ${F}_{i} = {σ}_{1} {Q}_{i} + {σ}_{2} {ρ}_{i}$
Among them, *σ*₁, *σ*₂ are all constants, *Qᵢ* is the content of the *i* raw material, and *ρᵢ* is the density of the *i* raw material;
Calculate the mixing time T using the following formula: $T = {σ}_{3} {Q}_{θ} + {σ}_{4}$
Among them, *σ*₃, *σ*₄ are constants, and 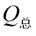 is the total content of all raw materials;
The motion path, gripping force and mixing time are integrated into execution instructions, and the robot arm recognizes the formula instructions and operates according to the corresponding execution instructions.
